# FASCICULE DE BREVET EUROPEEN

(11) **EP 3 372 297 B1**
(45) Date de publication et mention de la délivrance du brevet: **11.05.2022**
(21) Numéro de dépôt: 18160228.5
(22) Date de dépôt: 06.03.2018
(51) Int. Cl.: B01D 53/14, B01D 53/18, B01D 53/22, C12M 1/00, C10L 3/10

(54) **PROCEDE DE PURIFICATION DE GAZ, NOTAMMENT DE BIOGAZ POUR OBTENIR DU BIOMETHANE**
GASREINIGUNGSANLAGE, INSBESONDERE FÜR BIOGAS ZUR ERHALTUNG VON BIOMETHANGAS
METHOD FOR PURIFYING GASES, IN PARTICULAR BIOGAS, IN ORDER TO OBTAIN BIOMETHANE

(30) Priorité: 06.03.2017 FR 1751805
(43) Date de publication de la demande: 12.09.2018
(73) Titulaire: CentraleSupélec, 91190 - Gif sur Yvette (FR)
(72) Inventeur: THEOLEYRE, Marc-André, 75020 PARIS (FR); FOUGERIT, Valentin, 17740 SAINTE MARIE DE RE (FR); SMAOUI, Touhami, 78450 VILLEPREUX (FR); STAMBOULI, Moncef, 92290 CHATENAY-MALABRY (FR)
(74) Mandataire: Cabinet Bleger-Rhein-Poupon

(56) Documents cités:
- EP-A1- 2 714 243
- EP-A1- 3 047 894
- WO-A1-2012/096576
- WO-A1-2013/136310
- CN-A- 1 759 922
- US-A1- 2011 223 650
- US-A1- 2015 122 122
- US-A1- 2015 328 583

## Description

La présente invention entre dans le domaine de la séparation des gaz, et est applicable en particulier aux mélanges de gaz comprenant du CO₂ et du CH₄.

Cette invention permet par exemple la valorisation d'un gaz, plus particulièrement le biogaz, qui est produit lors de la méthanisation de matières organiques par des populations de micro-organismes.

Le processus de méthanisation permet d'aboutir à la production d'un biogaz composé notamment de méthane CH₄, dans une proportion comprise entre 50 et 70% en volume, de dioxyde de carbone CO₂,dans une proportion allant de 25 à 50% en volume, par rapport à la volume totale du biogaz produit, et de sulfure d'hydrogène H₂S, dans une concentration de 20 000 ppm maximum.

La filière de la méthanisation est en constant développement, notamment en France, et cela depuis de nombreuses années. Le gisement méthanisable a été évalué à 55,9 TWh (térawattheure) à l'horizon des années 2030.

Il est donc particulièrement intéressant de chercher à développer des process pour la valorisation du biogaz issu de la méthanisation de matières organiques et plus particulièrement l'utilisation de celui-ci dans le réseau de gaz naturel ou en tant que carburant.

Toutefois, l'injection de biogaz dans le réseau ne peut être effectuée qu'une fois que les impuretés et les gaz indésirables, notamment le CO₂ et l'H₂S, ont été éliminés du biogaz directement issu de la méthanisation.

Ainsi, pour augmenter sa valeur calorifique et répondre aux spécifications des opérateurs de réseau de gaz naturel pour une injection dans le réseau et/ou son utilisation comme carburant automobile, le biogaz issu directement du processus de méthanisation doit subir une épuration, en sorte de comporter, au final, une proportion de l'ordre de 92% et de préférence supérieure à 96% de CH₄ suivant la qualité du gaz distribué, On parle alors de « biométhane ».

La présente invention a donc pour objet un procédé visant à permettre une épuration efficace, notamment, du biogaz issu de la méthanisation, avec un rendement élevé, dans le but d'obtenir un biométhane d'une grande pureté et répondant aux caractéristiques requises, c'est-à-dire ayant plus de 92% de CH₄ en volume, par rapport au volume total de biométhane.

Dans l'état de l'art, différentes techniques ont été mises au point en sorte de permettre une purification du biogaz et d'obtenir un biogaz enrichi en méthane.

En particulier, certains documents de brevet décrivent l'utilisation de contacteurs à membranes pour l'épuration du biogaz.

Les contacteurs à membrane permettent, de manière générale, la séparation sélective d'une molécule particulière présente dans un fluide, dans le but de la valoriser, si celle-ci consiste par exemple en un composé aromatique d'intérêt, ou dans le but de l'éliminer, si ladite molécule est indésirable, notamment si celle-ci est toxique ou malodorante.

A cet effet, les contacteurs membranaires sont généralement constitués d'une membrane poreuse, matérialisant une interface séparant physiquement deux fluides ou plus, et dans laquelle les molécules sont séparées selon leurs affinités avec l'une ou l'autre des phases. Le déplacement de cette molécule dans la membrane s'effectue sous l'effet, généralement, d'un gradient de pression ou de concentration à travers la membrane.

Ces contacteurs membranaires ont déjà été utilisés dans le but d'éliminer le CO₂ du biogaz, et par conséquent pour obtenir un biométhane d'une grande pureté.

Ainsi, une phase gazeuse (biogaz) et une phase liquide (par exemple eau) sont confinées de chaque côté d'une membrane poreuse et hydrophobe, qui stabilise l'interface entre les deux phases, et les transferts entre phases s'effectuent par l'intermédiaire des pores de la membrane.

Dans le document de brevet EP 2 714 243, il est divulgué un procédé et une installation pour le traitement d'un gaz naturel contenant du méthane, afin d'éliminer les gaz indésirables de celui-ci, notamment le CO₂ et le H₂S.

Dans ce brevet, deux membranes sont arrangées en série dans un circuit, dans lequel circule de l'eau.

La première membrane, imperméable à l'eau et perméable aux gaz, est positionnée dans un module de dégazage et a pour fonction de permettre une élimination des gaz indésirables présents dans l'eau, par stripping via une pompe à vide, cette eau étant, au préalable, dessalée et présentant une conductivité inférieure à 500 pS/cm. En d'autres termes, le CO₂ dissous dans l'eau déminéralisée est éliminé de celle-ci dans le module de dégazage et passe de l'autre côté de la membrane.

L'eau dégazée passe ensuite dans un module d'échange des gaz, où se produit une absorption des gaz indésirables contenus dans du biogaz à traiter, celui-ci étant injecté à contrecourant de l'eau dégazée. Après cette étape, un gaz enrichi en méthane est obtenu. Ce gaz enrichi, s'il est qualifié, peut alimenter un réseau de gaz existant.

Ainsi, on a cherché ici, en diminuant au maximum la conductivité de l'eau, et en utilisant à cet effet de l'eau dessalée, à augmenter la solubilité du dioxyde de carbone, et donc la dissolution de celui-ci dans l'eau.

Cependant, l'ensemble des gaz indésirables présents à un moment donné dans l'eau (CO₂, O₂, H₂S...) se retrouvent donc en mélange au niveau du module de dégazage. Par le même mécanisme, une fraction de méthane, gaz dont on cherche à maximiser la valorisation, est mélangée dans ce flux avec une faible concentration. Les possibles valorisations de ce mélange de gaz avec une faible valeur énergétique est complexe et couteuse à mettre en œuvre.

On connait également, du document de brevet US 2011/223650, un procédé mettant en œuvre deux contacteurs en boucle, absorption désorption, ainsi que des enzymes de type anhydrase carbonique pour en améliorer les performances de séparation de CO₂ à partir d'un mélange de plusieurs gaz, les enzymes catalysant la conversion du CO₂ dissout en ions carbonates.

Dans le module d'absorption, le CO₂ peut être absorbé au travers d'un solvant chimique, c'est-à-dire qui absorbe le CO₂ par une réaction chimique, ledit solvant étant alors à base d'amine ou d'ammonium aqueux ou d'acides aminés. Un solvant physique (glycérol, polyéthylène glycol, éther de polyéthylène glycol, éthylène glycol diméthyl éther, etc.) peut également être utilisé, celui-ci absorbant le CO₂ sans mise en place d'une réaction chimique.

Les solvants chimiques comportent des composés dissous comme les amines, les sels d'aminoacides, les solutions de sels alcalins ou de sels phosphates ou de carbonates de potassium, qui absorbent le CO₂ par une réaction chimique et le consomment instantanément. La rapidité de cette réaction assure un potentiel chimique, dans la phase liquide, du gaz à absorber, par exemple le CO₂, particulièrement faible. Les solvants chimiques sont par conséquent généralement utilisés dans les procédés d'épuration de gaz, car la consommation rapide du gaz à absorber permet de maintenir une importante différence de potentiel qui accélère le transfert de matière et augmente la capacité d'absorption du solvant.

Dans un solvant physique, le transfert de matière, ou la quantité de CO₂ dissoute par unité de temps et de surface membranaire, est proportionnelle à la différence de potentiels chimiques du composé dans les phases liquide et gaz.

Dans le document de brevet WO 2012/096576, un mélange de gaz est mis en contact avec un liquide d'absorption au travers, par exemple, d'une colonne d'absorption ou d'un contacteur membranaire à une pression d'au moins 1 bar. Le liquide riche en gaz est ensuite régénéré au contact d'une membrane de désorption, avec application d'une dépression transmembranaire pour faciliter la désorption des gaz du liquide qui est, quant à lui, réutilisé.

D'une manière générale, dans les documents de l'état de la technique, on cherche à maximiser l'absorption, notamment de CO₂, à partir d'un mélange de gaz le contenant, en mettant en œuvre éventuellement des catalyseurs enzymatiques ou des solvants chimiques, afin d'obtenir, au final, un gaz purifié duquel a été extrait la quasi-totalité du dioxyde de carbone.

Il est d'ailleurs généralement admis que la solubilité des gaz dans un liquide, et notamment du dioxyde de carbone CO₂, a tendance à diminuer lorsque la salinité de ce liquide augmente. C'est la raison pour laquelle on cherche traditionnellement à diminuer le plus possible la salinité des liquides d'absorption comme, par exemple, dans le document de brevet EP 2 714 243 mentionné plus haut, où l'on utilise une eau déminéralisée dont la conductivité est faible.

En d'autres termes, la présence de sels dans un liquide ou un solvant diminue généralement la capacité d'absorption dudit liquide.

Les documents WO 2013/136310 A1, CN 1 759 922 A et US 2015/122122 A1 divulguent des procédés de séparation de dioxyde de carbone d'un biogaz au moyen d'une absorption avec de l'eau de mer dans des unités membranaires.

Cependant, les inventeurs ont pu déterminer que, inévitablement, lorsqu'on applique les techniques existantes à la purification, par exemple, de biogaz contenant notamment du dioxyde de carbone et du méthane, une proportion non négligeable de méthane est également dissoute dans le liquide d'absorption qui circule entre les modules d'absorption et de désorption, dans une boucle fermée et, généralement, pressurisée.

Le méthane, qui est dissous dans le liquide d'absorption est donc mélangé à d'autres impuretés et gaz indésirables, notamment le CO₂ et l'H₂S, est, par conséquent, extrêmement difficile à récupérer et à valoriser.

Ainsi, dans une démarche inventive, les inventeurs ont ici mis en évidence que, de manière surprenante, en utilisant, en tant que liquide d'absorption, un liquide ayant une salinité et, par conséquent, une conductivité élevées, ledit liquide comportant par exemple un sel de type NaCl (chlorure de sodium) ou KCl (chlorure de potassium), il est possible de diminuer substantiellement la proportion de gaz que l'on souhaite purifier, notamment de méthane CH₄, dissoute dans ledit liquide, tout en permettant, d'un autre côté, le maintien d'une absorption satisfaisante du gaz à éliminer, notamment le dioxyde de carbone CO₂.

Ainsi, les pertes en CH₄ par dissolution de celui-ci dans le liquide absorbant sont minimisées, tandis qu'on obtient, en parallèle, un biométhane d'une pureté suffisante pour une utilisation de celui-ci dans le réseau de gaz, avec une installation de conception relativement simple.

La présente invention concerne un procédé d'épuration d'un flux de biogaz entrant composé d'un mélange de gaz comprenant du méthane CH₄ à purifier et au moins un gaz indésirable à éliminer, comme du dioxyde de carbone CO₂ ou du sulfure d'hydrogène H₂S, ledit procédé comprenant au moins les étapes suivantes :
- lors d'une étape d'absorption, on met en contact, au travers d'une unité membranaire d'absorption comprenant au moins une membrane perméable aux gaz et imperméable aux liquides, le flux de biogaz entrant d'un côté de ladite membrane et un liquide absorbant circulant de l'autre côté de ladite membrane, et on obtient un liquide absorbant riche comportant le(s)dit(s) gaz indésirable(s) dissous et un flux de biométhane sortant, appauvri en gaz indésirable à éliminer, par exemple appauvri en CO₂;
- lors d'une étape de désorption, on met en contact ledit liquide absorbant riche avec une unité membranaire de désorption comprenant au moins une membrane, perméable aux gaz et imperméable aux liquides, et on élimine, par application d'une dépression transmembranaire, dudit liquide absorbant riche circulant d'un côté de ladite membrane, au moins une partie dudit (desdits) gaz indésirable(s) dissous, par passage au travers de ladite membrane vers l'autre côté de celle-ci, pour obtenir un liquide dégazé ;
- on recycle ledit liquide dégazé dans une étape d'absorption ultérieure ;

Dans ce procédé, ledit liquide absorbant comporte de l'eau et au moins un sel choisi parmi le chlorure de sodium NaCl, le chlorure de potassium KCl et le sulfate de sodium Na₂SO₄.

La concentration molaire en sel dans le liquide absorbant est comprise entre 1 et 2 mol/L et, plus avantageusement encore, cette concentration est de l'ordre de, ou égale à 1,5 mol/L.

Dans un mode de réalisation tout préférentiel du procédé de l'invention, le liquide absorbant comporte de l'eau et du chlorure de potassium KCl à une concentration comprise , entre 1 et 2 mol/L et, plus avantageusement encore, cette concentration est de l'ordre de, ou égale à 1,5 mol/L.

Dans un exemple de réalisation ledit liquide absorbant comporte, outre l'eau et au moins un sel choisi parmi NaCl, KC1, et Na₂SO₄, au moins un sel d'acide aminé, l'acide aminé étant choisi parmi la L-alanine, la L-proline, la L-glycine, la sarcosine et la taurine.

Toujours en combinaison avec un ou plusieurs sels NaCl/KCl/Na₂SO₄, et éventuellement avec un sel d'acide aminé, le liquide absorbant peut comporter une solution enzymatique comprenant une anhydrase carbonique combinée à au moins un régulateur de pH, ce dernier pouvant consister, par exemple en du Tris combiné avec de l'acide chlorhydrique HCl, ou en du phosphate ou en du carbonate de potassium.

Ledit liquide absorbant peut également comprendre une solution tampon choisie parmi le phosphate de monopotassium KH₂PO₄ ou le carbonate de potassium K₂CO₂.

La combinaison d'une solution d'acide(s) aminé(s) et/ou d'une solution enzymatique et/ou d'une solution tampon avec le liquide absorbant particulier mis en œuvre dans le procédé de l'invention est susceptible d'améliorer encore la sélectivité CO₂/CH₄ lors de l'étape d'absorption.

De manière intéressante, on fait circuler le liquide absorbant entre l'unité membranaire d'absorption et l'unité membranaire de désorption dans un circuit dans lequel ledit liquide est maintenu à une pression relative comprise entre 1 et 7 bars, de préférence entre 5 et 6 bars.

Dans un exemple de réalisation préférentiel, on maintient le flux de gaz qui arrive dans l'unité membranaire d'absorption à une pression relative comprise entre 10 mbars et 6 bars, de préférence à une pression relative comprise entre 4,5 et 5,5 bars, plus préférentiellement encore égale à 5 bars.

Avantageusement, on maintient le liquide absorbant à une pression supérieure d'au moins 0,2 bar à la pression du gaz à l'absorption.

En ce qui concerne l'étape de désorption, on applique, de préférence, une pression absolue, dans l'unité membranaire de désorption, et du côté de la membrane de sortie des gaz dissous, comprise entre 10 mbars et 1 bar.

La présente invention est également relative à une installation d'épuration d'un flux de gaz entrant, composé d'un mélange de gaz comprenant un premier gaz à purifier et au moins un gaz indésirable à éliminer, comme du dioxyde de carbone CO₂ ou du sulfure d'hydrogène H₂S, pour la mise en œuvre du procédé selon l'invention, comportant au moins une unité membranaire d'absorption comprenant au moins une membrane perméable aux gaz et imperméable aux liquides, ladite unité étant reliée, d'un côté de ladite membrane, à une entrée du flux de gaz et à une sortie du flux de gaz purifié, et, de l'autre côté de ladite membrane, à un circuit dans lequel circule un liquide absorbant le(s)dit(s) gaz indésirable(s), ledit circuit reliant l'unité membranaire d'absorption à une unité membranaire de désorption, laquelle comporte au moins une membrane perméable aux gaz et imperméable aux liquides apte à permettre un passage d'au moins une partie dudit (desdits) gaz dissous dans ledit liquide absorbant, ladite installation étant caractérisée par le fait que le liquide circulant dans ledit circuit comporte, au moins, de l'eau et au moins un sel choisi parmi le chlorure de sodium NaCl, le chlorure de potassium KCl et le sulfate de sodium Na₂SO₄, la concentration molaire en sel dans le liquide absorbant étant comprise entre 1 et 2

mol/ L.

Le procédé et l'installation d'épuration de gaz, notamment de biogaz, selon l'invention comportent de nombreux avantages.

En particulier, les inventeurs ont réussi à obtenir une élimination satisfaisante des gaz indésirables et des impuretés mélangés, à l'origine dans le biogaz, au méthane valorisable, tout en réduisant substantiellement les pertes en méthane, celles-ci se produisant inévitablement lors de la mise en œuvre de certaines techniques existantes. En d'autres termes, par la mise en œuvre du procédé de l'invention, on améliore le rendement de récupération du méthane CH₄ à partir du biogaz de départ, en comparaison avec les procédés actuels.

En outre, le procédé de l'invention est aisé à mettre en œuvre et les installations, pour l'application de ce procédé, peuvent consister en des petites unités ayant un coût d'investissement relativement peu élevé.

D'autres caractéristiques et avantages de l'invention ressortiront de la description détaillée qui va suivre des modes de réalisation non limitatifs de l'invention, en référence aux figures annexées dans lesquelles :
- la figure 1 représente, de manière schématisée, un mode de réalisation particulier et non limitatif d'une installation pour la mise en œuvre du procédé de l'invention ;
- la figure 2 représente, de manière schématisée, un autre mode de réalisation particulier d'une installation pour la mise en œuvre du procédé de l'invention avec une boucle intermédiaire de dégazage pour recycler une fraction des gaz dissous.

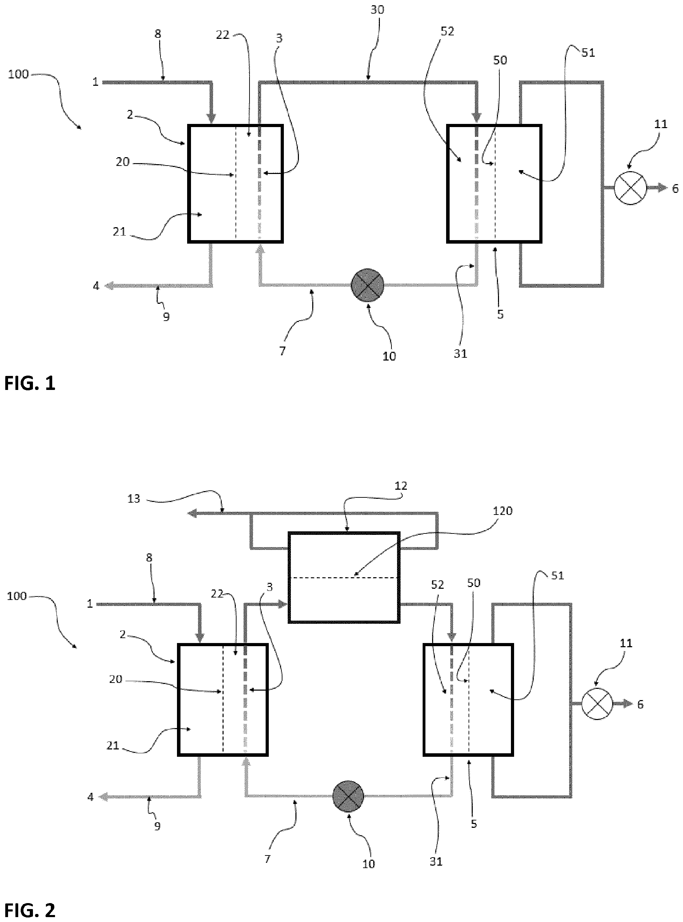

En référence à la figure 1, la présente invention concerne, particulièrement, un procédé d'épuration d'un flux de biogaz entrant 1. Ce dernier est composé d'un mélange de gaz et comprend, notamment, du méthane CH₄, que l'on cherche à purifier pour une valorisation ultérieure, et au moins un gaz indésirable, comme du dioxyde de carbone CO₂ ou du sulfure d'hydrogène H₂S, que l'on souhaite éliminer.

Le flux de biogaz entrant 1 peut être obtenu suite à la dégradation de matière organique par des micro-organismes. Ce biogaz 1 est composé en majorité de CH₄, dans une proportion généralement comprise entre 45 et 70% en volume par rapport au volume total de biogaz, et de CO₂, entre 25 et 50%.

Les autres gaz indésirables (H₂S, O₂, N₂...) ou impuretés présents également dans ce biogaz le sont dans des proportions relativement plus faibles.

La première étape du procédé de l'invention consiste en une étape d'absorption du ou des gaz indésirable(s) à éliminer par un liquide absorbant 3 et est mise en œuvre au travers, par exemple, d'une unité membranaire d'absorption 2.

Cette dernière comporte au moins une membrane 20, laquelle est hydrophobe et perméable aux gaz, tandis qu'elle est imperméable aux liquides. Cette membrane, microporeuse et hydrophobe, pourra par exemple être en polypropylène (PP), en polyfluorure de vinylidène (PVDF), ou bien encore en polytétrafluoroéthylène (PTFE).

Plus particulièrement, le flux de biogaz 1 arrive par l'intermédiaire d'une entrée 8 au niveau de ladite unité membranaire d'absorption 2, et se retrouve d'un côté 21 de ladite membrane 20 perméable aux gaz et imperméable aux liquides.

De l'autre côté 22 de ladite membrane 20 circule un liquide absorbant 3 dans un circuit 7, et de préférence à contrecourant par rapport aux flux de biogaz 1, comme illustré par exemple sur la figure 1.

Ainsi, dans la première étape d'absorption du procédé de l'invention, le(s) gaz indésirable(s), comme par exemple le CO₂ et l'H₂S, vont traverser la membrane et être dissous dans ledit liquide 3, en sorte d'obtenir, au niveau d'une sortie 9, un flux 4 de méthane CH₄ purifié, c'est à dire enrichi en méthane CH₄ et appauvri en gaz indésirable(s) par rapport au flux de biogaz entrant et comprenant, de manière particulièrement avantageuse, une proportion de CH₄ supérieure ou égale à 92%.

D'un autre côté, lorsque le(s) gaz indésirable(s) se dissolvent dans le liquide absorbant 3, on obtient un liquide absorbant riche 30 comprenant lesdits gaz.

Dans le procédé de la présente invention, de manière originale, le liquide absorbant 3 comporte au moins, de l'eau et un sel particulier.

Ainsi, selon l'invention, ledit liquide absorbant 3 comporte de l'eau et un sel choisi parmi le chlorure de sodium NaCl, le chlorure de potassium KCl, ou le sulfate de sodium Na₂SO₄.

Dans ce cas de figure, la concentration molaire en sel dans le liquide absorbant 3 est , selon l'invention, entre 1 et 2 mol/L et, plus avantageusement encore, cette concentration est de l'ordre de, ou égale à 1,5 mol/L.

De manière tout particulièrement avantageuse, le liquide absorbant 3 comporte de l'eau et du chlorure de potassium KCl à une concentration comprise entre 1 et 2 mol/L et, plus avantageusement encore, cette concentration est de l'ordre de, ou égale à 1,5 mol/L.

Il est également envisageable que le liquide absorbant comporte de l'eau et un mélange de plusieurs sels choisis parmi KCl, NaCl, et Na₂SO₄.

En d'autres termes, le liquide absorbant 3 est obtenu par dissolution de sel KCl, NaCl, ou Na₂SO₄ dans une solution constituée au moins d'eau, l'eau étant de préférence de l'eau osmosée.

Une eau osmosée est filtrée au travers d'une membrane d'osmose inverse, permettant de retenir les ions et les molécules de taille relativement importante, et présente une conductivité inférieure à 25 pS/cm, et plus préférentiellement encore inférieure à 20 pS/cm. Une telle eau est particulièrement avantageuse car elle notamment dépourvue d'ions Ca²⁺ et Mg²⁺ afin de limiter le risque de précipitation des ions carbonate dans les membranes.

Ainsi, le liquide absorbant mis en œuvre dans le procédé de l'invention est un solvant qualifié de solvant « physique », qui absorbe les composés gazeux indésirables par dissolution dans la phase liquide, à la différence des solvants dits « chimiques ».

Le liquide absorbant peut également comporter, en plus du sel tel que décrit précédemment, au moins un sel d'acide aminé, l'acide aminé étant choisi parmi la L-alanine, la L-proline, la L-glycine, la sarcosine et la taurine.

Dans ce cas de figure, la concentration molaire en sel d'acide aminé dans le liquide absorbant 3 est comprise entre 0,05 et 2 mol/L, avantageusement entre 0,5 et 1,5 mol/L.

Dans une autre mise en œuvre, ledit liquide absorbant 3 peut, outre l'eau et le sel, comporter au moins une solution enzymatique comprenant une anhydrase carbonique, une enzyme catalysant la réaction transformant du CO₂ dissous en ions hydrogénocarbonates HCO³⁻, cette enzyme étant combinée à un régulateur de pH.

Par exemple, ce régulateur de pH peut être soit un mélange de trishydroxyméthylaminométhane (également dénommé 2-amino-2-hydroxyméthyl-1,3-propanediol ou « Tris ») et d'acide chlorhydrique, soit du phosphate ou bien encore du potassium carbonate.

Dans ce cas de figure, la concentration massique en anhydrase carbonique dans le liquide absorbant 3 est comprise entre 0,1 et 500 mg/L, avantageusement entre 200 et 400 mg/L.

En ce qui concerne le régulateur de pH, celui-ci permet une régulation du pH comprise entre 4 et 10, avantageusement entre 5 et 9 et plus avantageusement encore entre 7 et 8.

L'anhydrase carbonique peut éventuellement être remplacée par un complexe synthétisé à partir d'un métal bivalent et présentant la même structure-réactivité qu'une anhydrase carbonique.

Enfin, ledit liquide absorbant 3 peut également comporter un mélange d'eau et de sel avec au moins une solution tampon choisie parmi le phosphate de monopotassium KH₂PO₄ ou bien le carbonate de potassium K₂CO₂.

Dans le premier cas de figure, la concentration massique en phosphate de monopotassium KH₂PO₄ dans le liquide absorbant 3 est comprise entre 2 et 22 % (m/V), avantageusement entre 10 et 20 %.

Dans le cas de l'utilisation du carbonate de potassium, la concentration massique est comprise entre 10 et 50 % (m/V), avantageusement entre 20 et 30 %.

Le liquide absorbant 3 mis en œuvre dans le présent procédé peut également comporter, dans un exemple de réalisation particulier, un mélange de différents composants tels que listés précédemment.

Ainsi, la présence d'un sel, en combinaison éventuelle avec un autre composant dans le liquide absorbant 3 confère une certaine conductivité audit liquide 3, dépendante de la concentration du sel et du composant additionnel éventuel dans ledit liquide 3.

Il a en effet été mis en évidence par la demanderesse que, de manière particulièrement surprenante, un tel liquide absorbant 3, composé d'eau et de sel type KCl et/ou NaCl et/ou Na₂SO₄, permet de minimiser, au cours de l'étape d'absorption des gaz, la dissolution dans le liquide 3 du gaz méthane CH₄ que l'on souhaite purifier, tandis que la dissolution des gaz indésirables, notamment le CO₂, dans ledit liquide 3 s'effectue toujours de manière satisfaisante pour une bonne élimination de celui-ci.

L'exemple 1 ci-dessous illustre la faible dissolution du méthane CH₄ dans le liquide absorbant 3 et l'absorption satisfaisante du CO₂ dans ledit liquide lorsque celui-ci est composé d'eau et de KCl.

L'exemple 2 ci-dessous illustre la conductivité ionique du liquide absorbant en fonction du sel présent dans ledit liquide et de la concentration en sel dans ce dernier.

Ainsi, grâce au procédé de l'invention, le rendement de récupération du méthane valorisable est substantiellement amélioré en comparaison avec les procédés de purification de biogaz existant dans l'état de la technique, dans lesquels une proportion importante de méthane est perdue par dissolution dans le liquide absorbant.

Dans le procédé de l'invention, suivant l'étape d'absorption, et une fois le dioxyde de carbone CO₂, et le (s) autre(s) gaz indésirable(s), dissous dans le liquide absorbant 3, on obtient un liquide absorbant riche 30.

Ce liquide absorbant riche 30 circule dans le circuit 7 jusqu'à une unité membranaire de désorption 5 dans laquelle, lors d'une étape de désorption, on va éliminer au moins une partie du ou des gaz indésirable(s) dissous, ces derniers traversant une membrane 50 que comporte ladite unité 5.

Cette membrane, microporeuse et hydrophobe, pourra par exemple être en polypropylène (PP), en polyfluorure de vinylidène (PVDF), ou bien encore en polytétrafluoroéthylène (PTFE).

On obtient alors un flux de gaz 6, ou « off-gaz » qui est destiné à être éliminé.

Tout comme pour la membrane 20 de l'unité d'absorption 2, la membrane 50 de l'unité de désorption 5 consiste en une membrane hydrophobe perméable aux gaz et imperméable aux liquides.

De manière plus précise, au cours de l'étape de désorption, le liquide absorbant riche en gaz indésirable(s) dissous 30 est amené au niveau de l'unité de désorption 5, d'un côté 52 de la membrane 50 que comporte ladite unité 5, et les gaz dissous vont être éliminés, au moins en partie, par passage de l'autre côté 51 de ladite membrane 50.

Suivant cette étape de désorption, on obtient alors un liquide absorbant dégazé 31, qui va être acheminé à nouveau, via le circuit 7, vers l'unité membranaire d'absorption 2 pour une nouvelle étape d'absorption de gaz indésirables.

En ce qui concerne la pression relative du liquide absorbant 3, 30, 31 circulant en boucle dans le circuit 7, celle-ci est avantageusement comprise entre 1 et 7 bars, de préférence entre 5 et 6 bars, en maintenant une perte de charge minimum pour limiter la consommation énergétique du procédé.

Une telle pression est préférentiellement maintenue au moyen d'une pompe 10, illustrée sur les figures.

Pour ce qui est à présent de l'étape d'absorption, le flux de biogaz 1 arrivant au niveau de l'unité membranaire d'absorption 2 est avantageusement maintenu à une pression relative comprise entre 10 mbars et 6 bars, cette pression étant plus avantageusement encore comprise entre 4,5 et 5,5 bars et, tout préférentiellement, égale à 5 bars.

Dans tous les cas, de manière préférentielle, la différence de pression entre la pression du liquide absorbant 3, 30, 31 circulant dans le circuit 7 et la pression du flux de biogaz 1 à l'absorption est supérieure ou égale à 0,2 bar.

Un tel différentiel de pression permet d'éviter le mélange des phases gaz et liquide par bullage du gaz dans le liquide.

Au niveau de l'étape de désorption à présent, on applique, préférentiellement, une pression absolue, du côté 51 de la membrane 50 par lequel les gaz indésirables dissous sont éliminés, comprise entre 10 mbars et 1 bar, par exemple au moyen d'une pompe à vide 11, en sorte de favoriser l'extraction desdits gaz, et d'obtenir un liquide absorbant dégazé 31 apte à être recyclé dans un nouveau cycle de purification d'un flux de biogaz 1, dans une étape d'absorption.

Plus préférentiellement encore, cette pression est comprise entre 80 et 90 mbars.

La présente invention est également relative à une installation 100 d'épuration d'un flux de gaz entrant composé d'un mélange de gaz comprenant un premier gaz à purifier et au moins un gaz indésirable à éliminer. Ledit flux entrant consiste préférentiellement en du biogaz 1 composé d'un mélange de plusieurs gaz comme déjà évoqué, notamment du CO₂ à éliminer et du CH₄ que l'on souhaite purifier. Ladite installation 100 est notamment illustrée sur la figure 1, et permet avantageusement la mise en œuvre des différentes étapes du procédé de l'invention.

L'installation 100 de l'invention comporte au moins :
- une entrée 8 du flux de gaz entrant, notamment du biogaz 1, et une sortie 9 du flux de gaz purifié, notamment du biométhane 4 ;
- entre ladite entrée 8 et ladite sortie 9, une unité membranaire d'absorption 2 comprenant au moins une membrane 20 perméable aux gaz et imperméable aux liquides ;
- un circuit 7 reliant l'unité membranaire d'absorption 2 à une unité membranaire de désorption 5 et dans lequel circule un liquide 3, 30, 31 absorbant lesdits gaz indésirables, ledit liquide comportant de l'eau et au moins un sel choisi parmi le chlorure de sodium NaCl, le chlorure de potassium KCl, et le sulfate de sodium Na₂SO₄;
- une unité membranaire de désorption 5 qui comporte au moins une membrane 50 perméable aux gaz et imperméable aux liquides apte à permettre un passage d'au moins une partie desdits gaz dissous 6 pour une élimination de ceux-ci.

Dans l'installation 100 de l'invention, le liquide circulant dans le circuit 7 peut également comporter, outre l'eau et le sel, au moins un sel d'acide aminé, l'acide aminé étant choisi parmi la L-alanine, la L-proline, la L-glycine, la sarcosine et la taurine, ou une solution enzymatique comprenant une anhydrase carbonique combinée à un régulateur de pH tel que du Tris combiné à du HCl ou du phosphate ou du potassium carbonate, ou encore une solution tampon choisie parmi le phosphate de monopotassium KH₂PO₄ ou le carbonate de potassium K₂CO₂.

De manière générale, les éléments qui ont été décrits pour le procédé de l'invention peuvent être appliqués à l'installation de l'invention.

Dans un exemple de réalisation intéressant, illustré sur la figure 2, l'installation 100 d'épuration d'un flux de biogaz 1 comporte en outre, entre l'unité membranaire d'absorption 1 et l'unité membranaire de désorption 5, une unité membranaire de dégazage intermédiaire 12, comportant au moins une membrane 120, ainsi qu'une boucle de recirculation 13.

Au moyen de cette unité complémentaire 12 et de cette boucle 13, qui permet de renvoyer le mélange de gaz extrait par cette unité 12 vers l'entrée 8 de l'installation 100, il est envisageable de réduire encore les pertes en méthane.

De préférence, au niveau de l'unité intermédiaire 12, aucun organe mécanique n'est utilisé pour faciliter le dégazage des gaz dissous. Le dégazage est contrôlé par le maintien d'une différence de pression entre les deux côtés de la membrane 120 de ladite unité 12.

La présente invention permet, de manière particulièrement avantageuse, de traiter un flux de biogaz par un procédé simple à mettre en œuvre et peu coûteux, et de récupérer le gaz méthane d'intérêt purifié, dans certains cas, à plus de 97%, tout en limitant les pertes en méthane au cours de l'étape critique d'absorption des gaz dans le liquide absorbant.

Le procédé et le dispositif de l'invention ont été décrits, dans ce qui précède, comme impliquant, lors des phases d'absorption et de désorption, une unité membranaire d'absorption et une unité membranaire de désorption, respectivement.

D'autres avantages et caractéristiques de l'invention apparaitront également à la lecture de l'exemple suivant, donnés à titre illustratif et non limitatif.

### Exemple 1 :

Dans des conditions opératoires similaires, la pureté en CH₄ du flux de biométhane sortant et le rendement de récupération du biométhane CH₄ ont été mesurés avec, en tant que liquide absorbant soit de l'eau osmosée, soit du chlorure de potassium, ce dernier ayant plus particulièrement été testé à des concentrations de 1,5 et 2,5 mol/L.

Les résultats obtenus sont repris dans le tableau 1 ci-dessous :

**Tableau 1 : synthèse des résultats obtenus (conditions d'adsorption: P gaz = 5 bars, P liquide = 5,5 bars, température = 15,5 °C +/- 0,5°C)**

| Solut° KCl | rapport | Désorption | BIOGAZ in | | Biométhane | | rendement |
|---|---|---|---|---|---|---|---|
| mole/l | G/L | P mb | débit NI/h | CH4 % | débit NI/h | CH4% | CH4 % |
| 0,0 | 1,0 | 85 | 90,0 | 60,5 | 40,8 | 94,6 | 70,9 |
| 1,5 | 1,0 | 79 | 90,0 | 60,0 | 45,1 | 97,3 | 81,3 |
| 2,5 | 1,0 | 80 | 90,0 | 60,4 | 47,9 | 97,3 | 85,7 |
| 0,0 | 1,7 | 86 | 153,0 | 60,3 | 99,0 | 84,6 | 90,8 |
| 1,5 | 1,7 | 78 | 153,2 | 60,3 | 96,0 | 94,9 | 98,6 |
| 2,5 | 1,7 | 76 | 153,0 | 60,7 | 96,5 | 93,0 | 96,7 |
| 2,5 | 1,7 | 83 | 94,9 | 60,2 | 54,0 | 96,5 | 91,2 |

Dans les différentes colonnes sont indiqués : le type de liquide absorbant utilisé dans l'exemple (avec ou sans KCl), le rapport entre le débit de flux de biogaz entrant et le débit de liquide absorbant circulant dans le circuit (G/L en Nl/L - normolitre (N1) de biogaz par Litre (L) de liquide absorbant), le débit de flux de biogaz entrant (G en Nl/h - normolitre (N1) de biogaz par heure), la pression de vide (P en mbar) lors de l'étape de désorption, la proportion de CH₄ (en %) dans le biogaz entrant, la pureté en méthane CH₄ du flux de biométhane sortant (Biométhane CH4 en %) et le rendement de récupération en CH₄ (en %) .

Les résultats obtenus, notamment avec un rapport G/L de 1,0 Nl/L et un débit G de 90 ou 153 Nl/h ou avec un rapport G/L de 1,7 Nl/L et un débit G de 95 ou 153 Nl/h, démontrent une amélioration systématique du rendement de récupération du méthane CH₄, lorsque le liquide absorbant comporte du KCl à une concentration de 1,5 mol/L ou de 2,5 mol/L (pas en accord complet avec l'invention) , en comparaison avec le rendement en CH₄ lorsque le liquide consiste simplement en de l'eau osmosée.

Ainsi, par la mise en œuvre du procédé de l'invention, notamment lorsque le liquide absorbant comporte du KCl, les pertes en CH₄ sont minimisées, en comparaison avec un procédé mettant en œuvre, en tant que liquide absorbant, de l'eau osmosée.

En outre, lorsque le liquide absorbant comporte du KCl, en ce qui concerne la pureté du flux de biométhane sortant, celle-ci est soit similaire à celle obtenue lorsque le liquide consiste en de l'eau, soit cette pureté est améliorée.

En effet, on remarque que, lorsque le rapport G/L est égal à 1,7 Nl/L et que le débit G est de 153 Nl/h, la pureté de CH₄ en sortie est de 84,6% lorsque le liquide absorbant consiste en de l'eau osmosée tandis que cette pureté atteint une valeur de l'ordre de 94,9% lorsque le liquide absorbant comporte du KCl à une concentration de 1,5 mol/L et une valeur de 93% lorsque la concentration molaire en KCL est de 2, 5 mol/L (pas en accord avec l'invention)

La qualité du biométhane pourrait être encore améliorée en installant plusieurs contacteurs en série.

### Exemple 2 :

La conductivité ionique des solutions mises en œuvre dans le procédé de l'invention en tant que liquide absorbant ont été mesurées de manière expérimentale, avec soit du NaCl, soit du KCl ou du Na₂SO₄ à différentes concentrations.

Les résultats obtenus sont repris dans le tableau 2 ci-dessous (les solutions selon l'invention ayant une concentration en sel de 1 à 2 mol/L) :

**Tableau 2 : synthèse des résultats obtenus pour la mesure expérimentale de la conductivité ionique (en mS/cm) à 20°C de solutions de NaCl, KCl, et Na₂SO₄ à différentes concentrations en sel de 0,2 à 0,5 mol/L**

| | | | | Valeurs expérimentales | | |
|---|---|---|---|---|---|---|
| Concentration | Concentration (g/L) | | | Conductivité ionique à 20°C (mS/cm) | | |
| mol/L | NaCl | KCl | Na₂SO₄ | NaCl | KCl | Na₂SO₄ |
| 0,2 | 11,7 | 14,9 | 28,4 | 18,1 | 23,9 | 27,1 |
| 0,5 | 29,2 | 37,3 | 71,0 | 41,3 | 51,4 | 51,7 |
| 1 | 58,4 | 74,6 | 142,0 | 70,0 | 87,0 | 74,0 |
| 1,5 | 87,7 | 111,8 | | 90,9 | 110,4 | |
| 2 | 116,9 | 149,1 | | 106,8 | 134,3 | |
| 2,5 | 146,1 | 186,4 | | 119,6 | 153,0 | |

Les conductivités mesurées lors des tests présentent des valeurs relativement importantes, en comparaison par exemple avec l'eau osmosée qui a une conductivité inférieure à 25 pS/cm, ou avec l'eau dessalée ou désionisée utilisée traditionnellement et dont la valeur indiquée ne dépasse pas les 500 pS/cm.

Ainsi, la conductivité d'un liquide absorbant comportant du NaCl dissous dans de l'eau osmosée, dans une concentration comprise entre 0,2 et 2,5 mol/L, et mesurée expérimentalement, est supérieure à 18 mS/cm.

Pour ce qui est d'un liquide absorbant avec du KCl dans une gamme de concentrations similaire, la conductivité molaire ionique expérimentale est supérieure à 23 mS/cm et celle d'un liquide comportant du Na₂SO₄ est supérieure à 27 mS/cm.

## Revendications

1. Procédé d'épuration d'un flux de gaz entrant (1) composé d'un mélange de gaz comprenant un premier gaz à purifier et au moins un gaz indésirable à éliminer, ledit flux de gaz entrant (1) consistant en un flux de biogaz comprenant du méthane CH₄ que l'on cherche à purifier et, en tant que gaz indésirables à éliminer, au moins du dioxyde de carbone CO₂ ou du sulfure d'hydrogène H₂S, ledit procédé comprenant au moins les étapes suivantes :
- lors d'une étape d'absorption, on met en contact, au travers d'une unité membranaire d'absorption (2) comprenant au moins une membrane (20) perméable aux gaz et imperméable aux liquides, le flux de biogaz entrant (1) d'un côté (21) de ladite membrane (2) et un liquide absorbant (3) circulant de l'autre côté (22) de ladite membrane (20), et on obtient un liquide absorbant riche (30) comportant lesdits gaz indésirables dissous et un flux de biométhane sortant (4) appauvri en gaz indésirables à éliminer ;
- lors d'une étape de désorption, on met en contact ledit liquide absorbant riche (30) avec une unité membranaire de désorption (5) comprenant au moins une membrane (50), perméable aux gaz et imperméable aux liquides, et on élimine, par application d'une dépression transmembranaire, dudit liquide absorbant riche (30) circulant d'un côté (52) de ladite membrane (50), au moins une partie dudit gaz dissous (6), par passage au travers de ladite membrane (50) vers l'autre côté (51) de celle-ci, pour obtenir un liquide dégazé (31) ;
- on recycle ledit liquide dégazé (31) dans une étape d'absorption ultérieure ;
**caractérisé par le fait que** ledit liquide absorbant (3, 30, 31) est un solvant physique, qui absorbe les composés gazeux indésirables par dissolution dans la phase liquide, ledit liquide absorbant (3, 30, 31) étant une solution aqueuse comportant de l'eau et un sel choisi parmi le chlorure de sodium NaCl, le chlorure de potassium KCl, et le sulfate de sodium Na₂SO₄, la concentration molaire en sel dans le liquide absorbant étant comprise entre 1 et 2 mol/L.

2. Procédé selon la revendication précédente **caractérisé par le fait que** la concentration molaire en sel dans le liquide absorbant est de l'ordre de, ou égale à 1,5 mol/L.

3. Procédé selon la revendication 1 dans lequel le liquide absorbant comporte de l'eau et du chlorure de potassium KCl à une concentration comprise entre 1 et 2 mol/L et, plus avantageusement encore, cette concentration est de l'ordre de, ou égale à 1,5 mol/L.

4. Procédé selon l'une quelconque des revendications précédentes **caractérisé par le fait que** ledit liquide absorbant (3, 30, 31) comporte en outre au moins un sel d'acide aminé, l'acide aminé étant choisi parmi la L-alanine, la L-proline, la L-glycine, la sarcosine et la taurine.

5. Procédé selon l'une quelconque des revendications précédentes **caractérisé par le fait que** ledit liquide absorbant (3, 30, 31) comporte en outre au moins une solution enzymatique comprenant une anhydrase carbonique combinée à au moins un régulateur de pH.

6. Procédé selon la revendication précédente **caractérisé par le fait que** régulateur de pH comporte du Tris en mélange avec de l'acide chlorhydrique HCl ou du phosphate ou du potassium carbonate.

7. Procédé selon l'une quelconque des revendications précédentes **caractérisé par le fait que** ledit liquide absorbant (3, 30, 31) comporte en outre au moins une solution tampon choisie parmi le phosphate de monopotassium KH₂PO₄ ou le carbonate de potassium K₂CO₂.

8. Procédé selon l'une quelconque des revendications précédentes **caractérisé par le fait que** l'on fait circuler du liquide absorbant (3, 30, 31) entre l'unité membranaire d'absorption (2) et l'unité membranaire de désorption (5) dans un circuit (7) dans lequel ledit liquide (3, 30, 31) est maintenu à une pression relative comprise entre 1 et 7 bars, de préférence entre 5 et 6 bars.

9. Procédé selon l'une quelconque des revendications précédentes, **caractérisé par le fait que** l'on maintient le flux de gaz (1) qui arrive dans l'unité membranaire d'absorption (2) à une pression relative comprise entre 10 mbars et 6 bars, de préférence à une pression relative comprise entre 4,5 et 5,5 bars, plus préférentiellement encore égale à 5 bars.

10. Procédé selon l'une quelconque des revendications 8 ou 9 **caractérisé par le fait que** l'on maintient le liquide absorbant (3, 30, 31) à une pression supérieure d'au moins 0,2 bar à la pression du gaz (1) à l'absorption.

11. Procédé selon l'une quelconque des revendications précédentes **caractérisé par le fait que** l'on applique une pression absolue, dans l'unité membranaire de désorption (5), du côté (51) de la membrane (50) de sortie des gaz dissous (6), comprise entre 10 mbars et 1 bar.

12. Installation (100) d'épuration d'un flux de gaz (1) entrant composé d'un mélange de gaz comprenant un premier gaz à purifier et au moins un gaz indésirable à éliminer, ledit flux de gaz entrant (1) consistant en un flux de biogaz comprenant du méthane CH₄ que l'on cherche à purifier et, en tant que gaz indésirables à éliminer, au moins du dioxyde de carbone CO₂ ou du sulfure d'hydrogène H₂S, pour la mise en oeuvre du procédé selon l'une quelconque des revendications précédentes, comportant au moins une unité membranaire d'absorption (2) comprenant au moins une membrane (20) perméable aux gaz et imperméable aux liquides, ladite unité (2) étant reliée, d'un côté (21) de ladite membrane (20), à une entrée (8) du flux de biogaz (1) et à une sortie (9) du flux de biométhane sortant (4) enrichi en gaz CH₄ à purifier et appauvri en gaz indésirables à éliminer, et, de l'autre côté (22) de ladite membrane (20), à un circuit (7) dans lequel circule un liquide (3, 30, 31) absorbant lesdits gaz indésirables, ledit circuit (7) reliant l'unité membranaire d'absorption (2) à une unité membranaire de désorption (5), laquelle comporte au moins une membrane (50) perméable aux gaz et imperméable aux liquides apte à permettre un passage d'au moins une partie desdits gaz dissous (6) dans ledit liquide absorbant (3, 30, 31), ladite installation (100) étant **caractérisée par le fait que** le liquide (3, 30, 31), circulant dans ledit circuit (7), est un solvant physique, qui absorbe les composés gazeux indésirables par dissolution dans la phase liquide, ledit liquide absorbant (3, 30, 31) étant une solution aqueuse comportant au moins, de l'eau et un sel choisi parmi le chlorure de sodium NaCl, le chlorure de potassium KCl, et le sulfate de sodium Na₂SO₄, la concentration molaire en sel dans le liquide absorbant étant comprise entre 1 et 2 mol/L.

13. Installation (100) d'épuration d'un flux de gaz (1) selon la revendication précédente **caractérisée par le fait que** ledit flux de gaz entrant (1) à traiter est amené à ladite unité membranaire d'absorption (2) à contrecourant avec ledit liquide absorbant (3, 30, 31).

## Patentansprüche

1. Verfahren zum Aufbereiten eines eintretenden Gasstroms (1), der aus einem Gasgemisch besteht, das ein erstes zu reinigendes Gas und wenigstens ein zu beseitigendes unerwünschtes Gas umfasst, wobei der eintretende Gasstrom (1) aus einem Biogasstrom besteht, der Methan (CH₄) umfasst, das insofern gereinigt werden soll, dass als unerwünschte Gase wenigstens Kohlenstoffdioxid (CO₂) oder Schwefelwasserstoff (H₂S) beseitigt werden, wobei das Verfahren wenigstens die folgenden Schritte umfasst:
- während eines Absorptionsschritts, Inberührungbringen des eintretenden Biogasstroms (1) auf einer Seite (21) der Membran (20) und einer Absorptionsflüssigkeit (3), die auf der anderen Seite (22) der Membran (20) zirkuliert, mittels einer Absorptionsmembraneinheit (2), die wenigstens eine Membran umfasst, die für Gase durchlässig und für Flüssigkeiten undurchlässig ist, und Erhalten einer angereicherten Absorptionsflüssigkeit (30), die die unerwünschten gelösten Gase aufweist, und eines austretenden Biomethanstroms (4), der an zu beseitigenden unerwünschten Gasen abgereichert ist;
- während eines Desorptionsschritts, Inberührungbringen der angereicherten Absorptionsflüssigkeit (30) mit einer Desorptionsmembraneinheit (5), die wenigstens eine Membran (50) umfasst, die für Gase durchlässig und für Flüssigkeiten undurchlässig ist, und Beseitigen durch Anwendung einer Transmembranvertiefung der angereicherten Absorptionsflüssigkeit (30), die auf einer Seite (52) der Membran (50) zirkuliert, wobei wenigstens ein Teil des gelösten Gases (6) mittels der Membran (50) zu deren anderer Seite (51) zum Erhalten einer entgasten Flüssigkeit (31) übergeht;
- Rückgewinnen der entgasten Flüssigkeit (31) in einem weiteren Absorptionsschritt; **dadurch gekennzeichnet, dass** die Absorptionsflüssigkeit (3, 30, 31) ein physikalisches Lösungsmittel ist, das die unerwünschten gasförmigen Verbindungen durch Auflösung in der flüssigen Phase absorbiert, wobei die Absorptionsflüssigkeit (3, 30, 31) eine wässrige Lösung ist, die Wasser und ein Salz aufweist, das aus Natriumchlorid (NaCl), Kaliumchlorid (KCl) und Natriumsulfat (Na₂SO₄) ausgewählt ist, wobei die Molarität des Salzes in der Absorptionsflüssigkeit zwischen 1 und 2 mol/l liegt.

2. Verfahren nach dem vorhergehenden Anspruch, **dadurch gekennzeichnet, dass** die Molarität des Salzes in der Absorptionsflüssigkeit in der Größenordnung von oder gleich 1,5 mol/l liegt.

3. Verfahren nach Anspruch 1, wobei die Absorptionsflüssigkeit Wasser und Kaliumchlorid (KCl) in einer Konzentration zwischen 1 und 2 mol/l aufweist und, noch vorteilhafter, diese Konzentration in der Größenordnung von oder gleich 1,5 mol/l liegt.

4. Verfahren nach einem der vorhergehenden Ansprüche, **dadurch gekennzeichnet, dass** die Absorptionsflüssigkeit (3, 30, 31) ferner wenigstens ein Salz einer Aminosäure aufweist, wobei die Aminosäure aus L-Alanin, L-Prolin, L-Glycin, Sarkosin und Taurin ausgewählt ist.

5. Verfahren nach einem der vorhergehenden Ansprüche, **dadurch gekennzeichnet, dass** die Absorptionsflüssigkeit (3, 30, 31) ferner wenigstens eine Enzymlösung aufweist, die eine Kohlensäureanhydrase umfasst, die mit wenigstens einem pH-Regulator kombiniert ist.

6. Verfahren nach dem vorhergehenden Anspruch, **dadurch gekennzeichnet, dass** der pH-Regulator Tris, das mit Salzsäure (HCl) oder Phosphat oder Kaliumcarbonat gemischt ist, aufweist.

7. Verfahren nach einem der vorhergehenden Ansprüche, **dadurch gekennzeichnet, dass** die Absorptionsflüssigkeit (3, 30, 31) ferner wenigstens eine Pufferlösung aufweist, die aus Monokaliumphosphat (KH₂PO₄) oder Kaliumcarbonat (K₂CO₃) ausgewählt ist.

8. Verfahren nach einem der vorhergehenden Ansprüche, **dadurch gekennzeichnet, dass** die Absorptionsflüssigkeit (3, 30, 31) zwischen der Absorptionsmembraneinheit (2) und der Desorptionsmembraneinheit (5) in einem Kreislauf (7) zirkuliert wird, in dem die Flüssigkeit (3, 30, 31) bei einem relativen Druck zwischen 1 und 7 bar, bevorzugt zwischen 5 und 6 bar, gehalten wird.

9. Verfahren nach einem der vorhergehenden Ansprüche, **dadurch gekennzeichnet, dass** der Gasstrom (1), der in die Absorptionsmembraneinheit (2) eintritt, bei einem relativen Druck zwischen 10 mbar und 6 bar, bevorzugt bei einem relativen Druck zwischen 4,5 und 5,5 bar, noch stärker bevorzugt von gleich 5 bar, gehalten wird.

10. Verfahren nach einem der Ansprüche 8 oder 9, **dadurch gekennzeichnet, dass** die Absorptionsflüssigkeit (3, 30, 31) bei einem Druck gehalten wird, der wenigstens 0,2 bar höher als der Druck des Gases (1) bei der Absorption ist.

11. Verfahren nach einem der vorhergehenden Ansprüche, **dadurch gekennzeichnet, dass** in der Desorptionsmembraneinheit (5) auf der Seite (51) der Austrittsmembran (50) der gelösten Gase (6) ein absoluter Druck angewendet wird, der zwischen 10 mbar und 1 bar beträgt.

12. Einrichtung (100) zum Aufbereiten eines eintretenden Gasstroms (1), der aus einem Gasgemisch besteht, das ein erstes zu reinigendes Gas und wenigstens ein zu beseitigendes unerwünschtes Gas umfasst, wobei der eintretende Gasstrom (1) aus einem Biogasstrom besteht, der Methan (CH₄) umfasst, das insofern gereinigt werden soll, dass als unerwünschte Gase, wenigstens Kohlenstoffdioxid (CO₂) oder Schwefelwasserstoff (H₂S), beseitigt werden, für das Durchführen des Verfahrens nach einem der vorhergehenden Ansprüche, die wenigstens eine Absorptionsmembraneinheit (2) aufweist, die wenigstens eine für Gase durchlässige und für Flüssigkeiten undurchlässige Membran (20) umfasst, wobei die Einheit (2) auf einer Seite (21) der Membran (20) mit einem Eintritt (8) des Biogasstroms (1) und an einem Austritt (9) des austretenden Biomethanstroms (4) mit CH₄-Gas angereichert ist, das gereinigt und an unerwünschten zu beseitigenden Gasen abgereichert werden soll, und auf der anderen Seite (22) der Membran (20) mit einem Kreislauf (7) verbunden ist, in dem eine Absorptionsflüssigkeit (3, 30, 31) zirkuliert, die die unerwünschten Gase absorbiert, wobei der Kreislauf (7) die Absorptionsmembraneinheit (2) mit einer Desorptionsmembraneinheit (5) verbindet, die wenigstens eine für Gase durchlässige und für Flüssigkeiten undurchlässige Membran (50) aufweist, die geeignet ist, ein Durchgehen wenigstens eines Teils der in der Absorptionsflüssigkeit (3, 30, 31) gelösten Gase (6) zuzulassen, wobei die Einrichtung (100) **dadurch gekennzeichnet ist, dass** die Flüssigkeit (3, 30, 31), die in dem Kreislauf (7) zirkuliert, ein physikalisches Lösungsmittel ist, das die unerwünschten gasförmigen Verbindungen durch Auflösung in der flüssigen Phase absorbiert, wobei die Absorptionsflüssigkeit (3, 30, 31) eine wässrige Lösung ist, die wenigstens Wasser und ein Salz aufweist, das aus Natriumchlorid (NaCl), Kaliumchlorid (KCl) und Natriumsulfat (Na₂SO₄) ausgewählt ist, wobei die Molarität des Salzes in der Absorptionsflüssigkeit zwischen 1 und 2 mol/l liegt.

13. Einrichtung (100) zum Aufbereiten eines Gasstroms (1) nach dem vorhergehenden Anspruch, **dadurch gekennzeichnet, dass** der zu behandelnde eintretende Gasstrom (1) im Gegenstrom mit der Absorptionsflüssigkeit (3, 30, 31) zu der Absorptionsmembraneinheit (2) geführt wird.

## Claims

1. Method for purifying an incoming gas flow (1) which consists of a gas mixture comprising a first gas to be purified and at least one undesirable gas to be removed, said incoming gas flow (1) consisting of a biogas flow comprising methane CH₄ which is intended to be purified and, as undesirable gases to be removed, at least carbon dioxide CO₂ or hydrogen sulfide H₂S, said method comprising at least the following steps:
- during an absorption step, by means of an absorption membrane unit (2) which has at least one membrane (20) that is permeable to gases and impermeable to liquids, the incoming biogas flow (1) on one side (21) of said membrane (2) and an absorbent liquid (3) which circulates on the other side (22) of said membrane (20) are brought into contact, and a rich absorbent liquid (30) comprising said dissolved undesirable gases and an outgoing biomethane flow (4) which is depleted of undesirable gases to be removed are obtained;
- during a desorption step, said rich absorbent liquid (30) is brought into contact with a desorption membrane unit (5) which has at least one membrane (50) that is permeable to gases and impermeable to liquids, and, by applying a transmembrane vacuum, at least part of said dissolved gas (6) is removed from said rich absorbent liquid (30) which circulates on one side (52) of said membrane (50) by passing through said membrane (50) toward the other side (51) thereof in order to obtain a degassed liquid (31);
- said degassed liquid (31) is recycled in a subsequent absorption step;
**characterized in that** said absorbent liquid (3, 30, 31) is a physical solvent which absorbs undesirable gaseous compounds by dissolution in the liquid phase, said absorbent liquid (3, 30, 31) being an aqueous solution comprising water and a salt chosen from sodium chloride NaCl, potassium chloride KCI, and sodium sulfate Na₂SO₄, the molar concentration of salt in the absorbent liquid being between 1 and 2 mol/L.

2. Method according to the preceding claim, **characterized in that** the molar concentration of salt in the absorbent liquid is around or equal to 1.5 mol/L.

3. Method according to claim 1, wherein the absorbent liquid comprises water and potassium chloride KCI at a concentration of between 1 and 2 mol/L and, even more advantageously, this concentration is around or equal to 1.5 mol/L.

4. Method according to any of the preceding claims, **characterized in that** said absorbent liquid (3, 30, 31) also comprises at least one amino acid salt, the amino acid being chosen from L-alanine, L-proline, L-glycine, sarcosine and taurine.

5. Method according to any of the preceding claims, **characterized in that** said absorbent liquid (3, 30, 31) also comprises at least one enzymatic solution having a carbonic anhydrase combined with at least one pH regulator.

6. Method according to the preceding claim, **characterized in that** the pH regulator comprises Tris mixed with hydrochloric acid HCI or phosphate or potassium carbonate.

7. Method according to any of the preceding claims, **characterized in that** said absorbent liquid (3, 30, 31) also comprises at least one buffer solution chosen from monopotassium phosphate KH₂PO₄ or potassium carbonate K₂CO₃.

8. Method according to any of the preceding claims, **characterized in that** absorbent liquid (3, 30, 31) is circulated between the absorption membrane unit (2) and the desorption membrane unit (5) in a circuit (7) in which said liquid (3, 30, 31) is kept at a relative pressure of between 1 and 7 bar, preferably between 5 and 6 bar.

9. Method according to any of the preceding claims, **characterized in that** the gas flow (1) which arrives in the absorption membrane unit (2) is kept at a relative pressure of between 10 mbar and 6 bar, preferably at a relative pressure of between 4.5 and 5.5 bar, even more preferably 5 bar.

10. Method according to either claim 8 or claim 9, **characterized in that** the absorbent liquid (3, 30, 31) is kept at a pressure at least 0.2 bar higher than the pressure of the absorption gas (1).

11. Method according to any of the preceding claims, **characterized in that** an absolute pressure of between 10 mbar and 1 bar is applied in the desorption membrane unit (5) on the side (51) of the membrane (50) where the dissolved gases (6) exit.

12. Facility (100) for purifying an incoming gas flow (1) which consists of a gas mixture comprising a first gas to be purified and at least one undesirable gas to be removed, said incoming gas flow (1) consisting of a biogas flow comprising methane CH₄ which is intended to be purified and, as undesirable gases to be removed, at least carbon dioxide CO₂ or hydrogen sulfide H₂S, for carrying out the method according to any of the preceding claims, comprising at least one absorption membrane unit (2) which has at least one membrane (20) that is permeable to gases and impermeable to liquids, said unit (2) being connected, on one side (21) of said membrane (20), to an entrance (8) for the biogas flow (1) and to an exit (9) for the outgoing biomethane flow (4) which is enriched with CH₄ to be purified and depleted of undesirable gases to be removed, and, on the other side (22) of said membrane (20), to a circuit (7) in which a liquid (3, 30, 31) circulates that absorbs said undesirable gases, said circuit (7) connecting the absorption membrane unit (2) to a desorption membrane unit (5) which comprises at least one membrane (50) that is permeable to gases and impermeable to liquids, which membrane is capable of allowing at least part of said dissolved gases (6) to pass into said absorbent liquid (3, 30, 31), said facility (100) being **characterized in that** the liquid (3, 30, 31) which circulates in said circuit (7) is a physical solvent that absorbs undesirable gaseous compounds by dissolution in the liquid phase, said absorbent liquid (3, 30, 31) being an aqueous solution comprising at least water and a salt chosen from sodium chloride NaCl, potassium chloride KCI, and sodium sulfate Na₂SO₄, the molar concentration of salt in the absorbent liquid being between 1 and 2 mol/L.

13. Facility (100) for purifying a gas flow (1) according to the preceding claim, **characterized in that** said incoming gas flow (1) to be treated is brought to said absorption membrane unit (2) countercurrently to said absorbent liquid (3, 30, 31).
